# EUROPEAN PATENT SPECIFICATION

(11) **EP 2 925 855 B1**
(45) Date of publication and mention of the grant of the patent: **28.02.2018**
(21) Application number: 13859211.8
(22) Date of filing: 24.10.2013
(51) Int. Cl.: C12N 1/20, A01C 1/06, A01H 5/10, A01N 63/02, A01P 21/00, C05F 11/08, C05F 3/00, C12N 11/00, A01H 3/00, C12R 1/07

(54) **PHOSPHATE SOLUBILIZING RHIZOBACTERIA BACILLUS FIRMUS AS BIOFERTILIZER TO INCREASE CANOLA YIELD**
PHOSPHATSOLUBILISIERENDE RHIZOBAKTERIEN DER SPEZIES BACILLUS FIRMUS ALS BIODÜNGEMITTEL ZUR ERHÖHUNG DER RAPSERNTE
RHIZOBACTÉRIE BACILLUS FIRMUS SOLUBILISANT LES PHOSPHATES UTILISÉE COMME BIOFERTILISANT POUR AUGMENTER LE RENDEMENT DE PRODUCTION DE CANOLA

(30) Priority: 30.11.2012 US 201261731706 P
(43) Date of publication of application: 07.10.2015
(73) Proprietor: Xitebio Technologies Inc., Winnipeg, Manitoba R2N 4A8 (CA)
(72) Inventor: BANERJEE, Manas Ranjan, Winnipeg Manitoba R2N 4A8 (CA)
(74) Representative: Franke, Dirk
(86) International application number: PCT/CA2013/050805
(87) International publication number: WO 2014/082167

(56) References cited:
- BANIK S ET AL: "EFFECT OF INOCULATION OF A PHOSPHATE-SOLUBILIZING PHYTOHORMONE PRODUCING BACILLUS-FIRMUS ON THE GROWTH AND YIELD OF SOYBEAN GLYCINE-MAX GROWN IN ACID SOIL OF NAGALAND INDIA", ZENTRALBLATT FUER MIKROBIOLOGIE, vol. 143, no. 2, 1988, pages 139-147, XP8180573, ISSN: 0232-4393
- ROD.IGUEZA, H ET AL.: 'Phosphate solubilizing bacteria and their role in plant growth promotion' BIOTECHNOLOGY ADVANCES vol. 17, no. 4-5, October 1999, pages 319 - 339, XP 002553030
- DATTA. M. ET AL.: 'Studies on the efficacy of a phytohormone producing phosphate solubilizing Bacillus finnus in augmenting paddy yield in acid soils of Nagaland.' PLANT AND SOIL vol. 69, no. 3, 1982, pages 365 - 373, XP008179079

## Description

### FIELD OF THE INVENTION

The present invention relates generally to the field of biofertilization and plant inoculants. More specifically, the present invention relates to application of naturally-occurring phosphate solubilizing rhizobacteria to increase canola growth, development and yield.

### BACKGROUND OF THE INVENTION

Phosphorus (P) is an essential nutrient for growth and development of plants, constituting up to 0.2% dry weight. Phosphorus is typically insoluble or poorly soluble in soils. Although the average P content of soils is about 0.05% (w/w), only 0.1% of the total P exists in plant accessible form (Illmer et al., 1995, Soil Biology and Biochemistry 27: 260-270). As a result, large amounts of soluble forms of P fertilizers are applied to meet the crop requirements to attain maximum production. Crops need more P than is dissolved in the soil solution to grow economically, therefore this P 'pool' must be replenished many times during the growing season. The ability of a soil to maintain adequate levels in the solution phase is the key to the plant available P status of a soil. However, the applied soluble forms of P fertilizers are easily precipitated into insoluble forms such as tricalcium phosphate [Ca₃(PO4)₂], ferric phosphate (FePO₄) and aluminum phosphate (AlPO₄) (Achal et al., 2007, Soil Biology and Biochemistry 39: 695-699). Inorganic P is negatively charged in most soils. Because of its particular chemistry, P reacts readily with positively charged calcium (Ca), iron (Fe) and aluminum (Al) ions to form relatively insoluble substances. When this occurs, the P is considered "fixed" or "tied up". Thus, P is fixed in the soil by locking itself and rendered unavailable to plants. This is also the case in western Canada as the majority of western Canadian soils are neutral to alkaline pH and calcium phosphate minerals are the dominant inorganic precipitates. It has been found that approximately 75-90% of applied P fertilizer is precipitated by Ca, Fe and Al cations. These insoluble forms are not efficiently taken up by the plants and thus lead to an excess application of P fertilizer to crop fields (Khan et al., 2007, Agronomy and Sustainable Development 27: 29-43). The application of P fertilizer initially adds to the levels of available P already present in soil. A portion of applied P is used in the year of application (10-30%), and the remaining unused P reverts to forms of soil P which become increasingly less available to the plant.

As per Goldstein et al. (1993, Bio/Techhnology 11: 1250-1254) the unavailable phosphates built up in soils are adequate to sustain maximum crop yields globally for about 100 years. Moreover, excess P application also enhances the potential for P loss to surface waters through overland or subsurface flow that accelerates freshwater eutrophication. This is the process in bodies of water of stimulating algal growth which ultimately die and decay in the water, and deplete available oxygen. The reduced oxygen levels ultimately result in reduced higher-order aquatic plant and animal populations. The P that can contribute to the enrichment of water bodies, and hence lead to eutrophication, is a combination of P that is attached to soil particles less than 0.45 µm in size that are transported during soil movement. The risk of P losses to the environment through surface runoff is greatest on sloping lands, and where fertilizer is surface applied and then followed by rainfall or irrigation. Manitoba fresh water lakes are one such examples of eutrophication. Eutrophication of most fresh water around the world is accelerated by P inputs and therefore, P is often the limiting element, and its control is of prime importance in reducing the accelerated eutrophication of fresh waters (USDA, 2003 in Agricultural phosphorus and eutrophication, Second Edition, (Sharpley, Daniel, Sims, Lemunyon, Stevens and Parry, eds), ARS-149, pp 38).

Phosphorus is vital for stable food production systems and for buffering against climate change impacts on soil. This is important for both crop and livestock production (AIC, 2010, Agriculture Institute of Canada Notes, June 3, issue 22). It is not always realized that phosphate is a scarce raw material, probably the most critical one. High quality P reserves are diminishing and the cost of fertilizers is escalating rapidly. Global reserves of phosphate (with >20% P₂O₅ content) seem to be in the range of 10000 million tonnes. With a future annual consumption of 40-50 million tonnes of P₂O₅ these reserves would last less than 200 years (FAO, 2006, FAO (Food and Agriculture Organization) Fertilizer and Plant Nutrition Bulletin 16, pp 348). Therefore, use of phosphate fertilizers need to be as judicious as possible and nutrient use efficiency of the phosphate fertilizers are required to be improved considerably. This is a particularly relevant and important topic in the light of the increasing global population as well.

Phosphorus occurs in soil in both organic and inorganic forms that differ greatly in terms of their solubility and mobility. Phosphorus applied through mineral fertilizers is in inorganic forms of varying solubility. Even at optimal rates, the use of mineral fertilizers and organic manures can lead to a buildup of soil P over time. Plants take up inorganic phosphate in two soluble forms: monobasic (H₂PO4⁻) and dibasic (HPO4²⁻) ions (Vessey, 2003, Plant and Soil 255: 571-586; Banerjee et al., 2006 in Hand Book of Microbial Biofertilizers (Rai, ed) pp 137-181). Some soil microorganisms are able to solubilize these insoluble P forms through the process of organic acid production, chelation, ion exchange reactions and polymeric substances formation, and make P available to plants (Vessey, 2003; Delvasto et al., 2006, Indian J. Mar. Sci. 29: 48-51; Chang and Yang, 2009, Bioresour. Technol. 100: 1648-1658). Seed or soil inoculations with phosphate solubilizing microbes have largely been used to improve crop growth and production by solubilization of fixed and applied phosphates (Nautiyal et al., 2000, FEMS Microbiology Letters 182: 291-296; Adesemoye and Kloepper, 2009, Applied Microbiology and Biotechnology 85: 1-12). Phosphate solubilizing bacteria play a role in P nutrition by enhancing its availability to plants through release from inorganic and organic soil P pools by solubilization and mineralization. The principal mechanism in soil for mineral phosphate solubilzation is the lowering of soil pH by microbial production of organic acids and mineralization of organic P by acid phosphatase enzyme (Sharma et al., 2011, J. Microbiol. Biotech. Res. 1(2): 90-95). The existence of microorganisms able to solubilize various forms of phosphates has been reported frequently elsewhere (e.g., Khan et al., 2009, J. Agric. Biol. Sci. 1(1): 48-58; Chakkaravarthy et al., 2010, J. Biol. Sci. 10(6): 531-535) but the success of utilizing the P-solubilizing plant growth promoting rhizobacteria (PGPR) as a commercial bioinoculant in different agroclimatic conditions in Canada (as well as USA) is yet to be determined appropriately. The PGPR and rhizosphere bacteria are free-living soil organisms that benefit plant growth by different mechanisms (Glick, 1995, Canadian Journal of Microbiology 41: 109-117). The ability of microorganisms to solubilize phosphorus is considered to be one of the most important traits associated with plant P nutrition (Chen et al., 2006, Applied Soil Ecology 34: 33-41). Hence, a biological seed treatment or bioinoculant with suitable formulation with naturally occurring P-solubilizing PGPR has tremendous potential to enhance production in prairie agronomic crops like canola (*Brassica napus* L.) with lower input cost (e.g., Banerjee and Yesmin, 2000, Agronomy Abstracts, Annual Meeting, Soil Science Society of America, pp 257). Canola is a major cash crop of the Canadian prairies (approx. 20 million acres) and any improvement in their yield potential would be substantial to the Canadian farmers as well as the economy. Thus, enhancing the production of canola with consistent performing phosphate solubilizing PGPR bioinoculant could be huge. In general, biological fertilization or biofertilizer is based on the use of natural inputs like microorganisms (e.g., bacteria, fungi) and are used to improve soil nutrient availability, produce growth stimulant for plant, improve soil stability, recycle nutrients, promote mycorrhiza symbiosis and develop bioremediation process in soil (Carvajal-Munoz and Carmona-Garcia, 2012, Livestock Research for Rural Development 24(3): pp 1-7). Hence, the naturally-occurring phosphate solubilizing PGPR could have a real potential to be used as a canola biofertilizer to enhance the canola production in western Canada and elsewhere.

US Patent 5,503,652 teaches the isolation of strains that are capable of promoting root elongation in plants.

US Patent 5,503,651 teaches the use of PGPR strains in promoting growth of cereals, oil seeds and maize based on the chemotactic and root-colonizing capabilities of the strains.

US Patent 6,406,690B1 teaches the use of *Bacillus firmus* strain CNCN I-1582 and *Bacillus cereus* strain CNCN I-1562 to control plant pathogenic nematodes.

US Patent 20060083725A1 teaches the use of a *Bacillus firmus* strain to supress bacterial and fungal disease in roots and tubers.

CN Patent 1355292-A and 1142268-C teach the use of *Bacillus firmus* strain BC9 KCCM 10865P that are highly able to remove nasty odors and heavy metals of livestock wastewater and food waste.

US Patent 5,244,658 teaches isolation of *Pseudomonas cepacia* strain which controls root rot caused by Aphanomyces fungus in pea.

US Patent 4,849,008 teaches applying *Pseudomonas* to the roots, plants, seeds, seed pieces or soil of root crops for enhancing the yield of root crops.

US Patent 6,194,193 teaches the use of a formulation for enhancing plant growth which comprises a mixture of *Bacillus* and *Paenbacillus* strains which produce phytohormones.

US Patent 5,589,381 teaches the isolation of a biocontrol element comprising a *Bacillus licheniformis* strain which controls *Fusarium* seedling blight in corn.

US Patent 6,232,270 teaches the treatment of plants by a composition containing agriculturally active ingredient and enhancer additive containing a pure culture of bacteria selected from genus Bacillus or soil bacteria, and the added culture may be added as cells, spores or suspensions.

DE Patent application 20110257009 teaches a method curatively controlling phytopathogenic fungi of plants using a composition of dithiino-tetracarboximide and agriculturally beneficial biological control agent.

CA Patent 2720739 teaches an agriculturally stable aqueous formulation comprising of bacterial spores, fungal spores and an organic solvent.

US Patent 8,008,545 teaches a method of production of fine chemicals like amino acids, vitamins, carbohydrates, fatty acids and carotenoids in a microorganism, plant, plant cell and plant tissue.

The basic requirement of profitable crop production is to produce an agronomic yield that can maximize net returns. Even the highest yield would not be of interest if its production were not cost-effective. Most farmers would like to maximize the net gains from whatever investment they can make in inputs. However, they also realize that top profits are possible only with optimal investment along with the correct decisions about the proper and innovative inputs (like P-solubilizing bioinoculant).

There has been much research conducted on the use of organisms to increase P availability in soils by "unlocking" P present in otherwise sparingly soluble forms. These microbes help in the solubilization of P from phosphate rock and other insoluble forms of soil P, and in the process decreasing their particle size, reducing it to nearly amorphous forms. Datta et al. (1982, Plant and Soil 69: 365-373) reported of a phytohormone producing phosphate solubilizing *Bacillus firmus* in augmenting paddy yield in acid soils of Nagaland. deFreitas et al. (1997, Biology and Fertility of Soils 24: 358-364) showed using phosphate solubilizing bacteria enhances the growth and yield of canola but P uptake in canola was not augmented. In addition to bacteria, the fungus *Penicillium bilaii* has been shown to increase P availability from native soils and phosphate rock sources in calcareous soils (Kucey, 1983, Canadian Journal of Soil Sceince 63: 671-678; Kucey and Leggett, 1989, Canadian Journal of Soil Science 69: 425-432). In fact, there is only one product in North America that contains a single action fungal P-solubilizer making it difficult for the inoculant to cope with the environmental stresses and be competitive. Bacteria are more effective in P solubilisation than fungi (Alam et al., 2002, Intl. J. Agric. Biol. 4: 454-458). Among the whole microbial population in soil, P solubilizing bacteria constitute 1-50%, while P solubilizing fungi are only 0.1-0.5% in P solubilisation potential (Chen et al., 2006, Appl. Soil Ecol. 34: 33-41). In addition, fungal inoculants are generally less competitive compared to bacterial inoculants and fungal spores are also not easy to mass-produce. However, in some other countries (such as India, Taiwan) the P-solubilizing bacteria are becoming popular (Zaidi et al., 2009, Acta Microbio Immunol Hung 56 (3): 263-284; Chang and Yang, 2009, Bioresour Technol 100 (4): 1648-1658; Ekin, 2010, African Journal of Biotechnology 9 (25): 3794-3800), ranking next in importance to the nitrogen-fixing *Rhizobium* inoculants, and usually more than one type of organism is used while preparing the P-solubilizing biofertilizer. The present invention introduces not only pure culture but also a consortia based approach with multiple strains of P-solubilizing bacteria. It shows the feasibility of potential use of mixed bacteria to form synergistic consortia and will create greater competitive ability to perform consistently under different growing conditions (Yesmin and Banerjee, 2001, in Proceedings of Saskatchewan Soils and Crops Workshop 2001, pp 314-319).

Although the pure culture or consortia culture inoculant may divulge enormous possibilities for canola, for a biological inoculant to be commercially effective, it must be mass-produced efficiently and formulated into a cost-effective, uniform, and readily applicable form (Walter and Paau, 1997 in Soil Microbial Ecology: Applications in Agricultural and Environmental Management, Metting, Jr., (ed.), pp. 579-594). Much of the studies have done for identifying the possible microbiological active, yet little has been investigated on these particular aspects. The benefit of microbial inoculation for greater crop production is significantly impacted by the number of live cells introduced into soil (Duquenne et al., 1999, FEMS Microbiology Ecology 29: 331-339). Furthermore, biological activity of microbes may also decline rapidly with handling and storage procedure. Daza et al. (2000, Soil Biology and Biochemistry, 32: 567-572) evaluated a peat and a perlite-based inoculant, and showed that sucrose adhesive along with the perlite carrier gave better viability of bacteria on seeds. A key limitation to successfully commercializing beneficial microorganisms is overcoming difficulties in creating a viable, cost-effective, and user-friendly final product (Xavier et al, 2004 in Crop Management Network, Symposium Proceedings Great Plains Inoculant Forum, Saskatoon, Saskatckhewan, pp. 1-6). Thus, it is critical to ascertain the length of bacterial survivability once the bacterial seed treatment is done and to attain the required level of bacterial population for the inoculant to be efficacious.

In most Canadian canola acres, treated seeds are regularly used as a critical component to control plant diseases. These fungicides/insecticides (e.g. Helix Xtra, Prosper FX, etc.) formulated as a suspension are used as seed treatments to control pre-emergence damping off, seed decay and other soil-borne diseases. It is anticipated that the bacterial cultures may not be alive with these pesticides at the recommended doses due to their high toxicity towards the bacteria (Yesmin and Banerjee, 2000, Agronomy Abstracts, Annual Meeting, Soil Science Society of America, pp 257; Yesmin and Banerjee, 2001). Our innovative approach of P-solubilizing formulations will allow high survivability of introduced bacteria across various environmental constrains, thereby ensuring higher yield and greater productivity. This will also resolve issues related to seed treatment chemicals and will give the farmers flexibility in terms of choice of seed treatment chemicals. Ensuring high survivability of these bacteria will eventually ensure greater P availability and lower input of costly P fertilizer for the crops.

### SUMMARY OF THE INVENTION

According to a first aspect of the invention, there is provided a bacterial culture consisting of: a biologically pure culture of XSB375, identified as *Bacillus firmus* (ATCC# PTA-120309).

According to a second aspect of the invention, there is provided a bacterial culture consisting of: a biologically pure culture of XSB375, identified as *Bacillus firmus,* (ATCC# PTA-120309) capable of phosphate solubilization.

According to a third aspect of the invention, there is provided a method of increasing plant growth and yield comprising:
inoculating a soil environment or potting mixture with a biologically pure culture of XSB375, identified as *Bacillus firmus* (ATCC# PTA-120309) and growing a plant in said soil environment or potting mixture.
According to a fourth aspect of the invention, there is provided a composition of matter comprising one phosphate solubilizing PGPR XSB375, identified as *Bacillus firmus* (ATCC# PTA-120309); and
an agriculturally compatible carrier.

According to a fifth aspect of the invention, there is provided a composition of matter comprising one phosphate solubilizing PGPR XSB375, identified as *Bacillus firmus,* (ATCC# PTA-120309), in liquid suspension. Also disclosed is a seed coated with phosphate solubilizing PGPR XSB375, identified as *Bacillus firmus* (ATCC# PTA-120309). Also disclosed is a seed application or soil application or foliar application or post emergence application with a phosphate solubilizing PGPR XSB375, identified as *Bacillus firmus* (ATCC# PTA-120309). Also disclosed is a phosphate solubilizing PGPR XSB375, identified as *Bacillus firmus,* (ATCC# PTA-120309), to be utilized as a biofertilizer.

### BRIEF DESCRIPTION OF THE DRAWINGS

Figure 1: PGPR strain XSB375 showing clearing zone through phosphate solubilization. Thus, the strain is a phosphate solubilizing rhizobacteria.
Figure 2: Canola seed emergence in soil was depicted after canola seeds inoculated with PGPR strain XSB375 compared to control up to 10 days. The bacterial strain enhanced the canola seed emergence in comparison to uninoculated control.
Figure 3: Growth of canola fine root hairs was depicted after canola seeds inoculated with PGPR strains XSB375 compared to control at 3 days. The strain enhanced the root growth in comparison to control.
Figure 4: Canola root and hypocotyl growth at 7 days was depicted for seeds inoculated with PGPR strain XSB375 compared to control in growth pouch. The root and hypocotyls growth considerably enhanced due to the inoculation of strain XSB375 in comparison to control.
Figure 5: Canola root and hypocotyl growth at 7 days was measured for seeds inoculated with PGPR strain XSB375 compared to control in growth pouch. The root and hypocotyls growth considerably enhanced due to the inoculation of strain XSB375 in comparison to control.
Figure 6: Viability of PGPR strain XSB375 in liquid suspension. The number of bacterial count showed that it is well over 1x10⁹ cfu/ml at 244 days. Thus, bacterial survivability in the liquid suspension would be over 244 days.
Figure 7: Canola yields in Regent, ND; Hazen, ND; and Bottineau, ND field trial sites in year 2012. The PGPR XSB375 treatment increased canola yield by 19.4% compared to the control in Regent, ND site. The PGPR XSB375 treatment increased canola yield by 14.6% compared to the control in Hazen, ND site. The PGPR XSB375 treatment increased canola yield by 6.6% compared to the control in Bottineau, ND site. Thus, the PGPR treatment XSB375 on an average increased canola yield by 13.5% compared to the uninoculated control.

### DESCRIPTION OF THE PREFERRED EMBODIMENTS

Unless defined otherwise, all technical and scientific terms used herein have the same meaning as commonly understood by one of ordinary skill in the art to which the invention belongs.

### DEFINITIONS

As used herein, "biologically pure" refers to a culture wherein virtually all of the cells present are of the selected strain.

As used herein, "inoculating" refers to introducing at least one bacterium into a medium, for example, a liquid medium, granular, peat powder, seed or a soil environment.

As used herein, "PGPR" or "plant growth promoting rhizobacteria" refers to plant beneficial isolates which inhabit the area surrounding plant roots.

As used herein, "soil environment" refers to the soil in which a plant is grown or is growing.

As used herein, "XSB375" refers to *Bacillus firmus,* deposited with ATCC under deposit number PTA-120309 on April 23, 2013.

As will be appreciated by one of skill in the art, as used herein, "phosphate solubilizing PGPR" refers to beneficial bacterial cultures and isolates as well as cell extracts (extracellular or intracellular) or enzymes purified or genes isolated therefrom or DNA isolated therefrom derived therefrom capable of solubilizing phosphate, promoting phosphorus uptake, improving plant growth, development and/or improving plant yield.

Described herein is the isolation and identification of a phosphate solubilizing plant growth promoting rhizobacteria (PGPR): XSB375, identified as *Bacillus firmus* (ATCC# PTA-120309). The PGPR is capable to solubilize phosphate that enhance plant available phosphorous. The PGPR XSB375 enhances seed germination, early emergence, plant vigor, root and shoot growth, and crop yield. This increased crop yield is attributed not only from the greater phosphate solubilzation, making more available phosphorus to plant but also making other essential plant nutrients more available to the plant. The application of this phosphate solubilizing PGPR can be done as liquid suspension or as solid materials applied to soil, potting mixture, seeds, seed pieces, seedlings, foliage, carrier materials, roots and planting soil. For example, the phosphate solubilizing PGPR may be coated onto a seed or seed piece, may be applied as a powder, may be applied as a liquid, may be applied foliar or as a suspension to a soil environment or may be mixed into a soil environment prior to use of the soil environment for planting.

Accordingly, in one aspect of the invention, there is provided a biologically pure bacterial culture of XSB375 (*Bacillus firmus*).

In another aspect of the invention, there is provided a method of increasing plant growth and/or yield comprising: inoculating with phosphate solubilizing PGPR XSB375 (*Bacillus firmus*) in soil environment or potting mixture; and growing a plant in said soil environment or potting mixture.

As discussed herein, in some embodiments, the soil environment or potting mixture includes phosphorus as a fertilizer. Specifically, the presence of PGPR XSB375 will result in greater and more efficient uptake of the phosphorus by the plants. That is, the application of XSB375 to the soil environment or potting mixture accomplishes at least one of the following: promote P-uptake in a plant; increase solubilisation of phosphate to H₂PO₄⁻ within the soil environment; increase solubilisation of phosphate to HPO₄²⁻ in the soil environment; alleviate a P-deficiency in a plant; alleviate P-deficiency in P-deficient soil fertilized with P fertilizer; promote uptake of macronutrients in a plant; and promote uptake of micronutrients in a plant. As will be appreciated by one of skill in the art, other benefits may also be observed and/or attained by application of PGPR XSB375 to the soil environment or potting mixture. Such additional benefits will be readily apparent to those of skill in the art.

As will be appreciated by one of skill in the art, the plant may be any suitable plant, for example, a plant which would benefit from increased P uptake, as discussed above.

In some embodiments, the plant is selected from the group consisting of oil seeds, legumes, non-legumes, cereals, root plants, vegetables, grasses, forage, turfs, fruits and flowering plants.

Alternatively, in other embodiments, the plant is selected from the group consisting of canola, soybean, sunflower, flax, hemp, wheat, barley, corn, millet, oat, pea, lentil, beans, peanut, alfalfa, clover, sugar beets, potatoes, carrots, cabbages, tomatoes, radishes, capsicum, cucumber, grasses, turf, cantaloupe, melon, fruits and flowers. The flowers may be selected from the group consisting of rose, chrysanthemum, lily and gerbera.

As discussed herein, XSB375 may be mixed into the soil environment or potting mixture. For example, XSB375 may be applied to the soil environment as a liquid suspension, for example, on a plant, on a post emergent plant, on a seed, on a seedling, or on carrier materials, as discussed herein.

In some embodiments, XSB375 is applied to the soil environment as a coating on a seed, as discussed below.

In other embodiments, there is provided a composition of matter comprising one phosphate solubilizing PGPR XSB375 (*Bacillus firmus*); and an agriculturally compatible carrier.

As will be appreciated by one of skill in the art, the carrier may be is a seed wherein XSB375 is coated onto the seed. The seed may be coated with peat or clay or mineral or vermiculite or polymer.

Alternatively, the carrier may be a liquid suspension, for example, an agriculturally compatible oil.

In other embodiments, the carrier may be a granular material, for example, peat or clay or perlite granules or mixture thereof.

As will be appreciated by one of skill in the art, XSB375 may be co-applied, either simultaneously or in serial with other suitable treatments applied to the soil environment to further improve plant growth. For example, XSB375 may be applied with another PGPR strain or with a compatible herbicide or fungicide or insecticide or seed treatment.

In the examples, the plant is canola which, as discussed above, is a high-phosphorus requiring crop. However, as will be apparent to one knowledgeable in the art, as discussed above, the phosphate solubilizing PGPR described herein may be used to promote growth of any suitable plant, for example cereals, for example corn, wheat, barley, oat and the like; oil seeds, for example canola, flax, hemp, sunflower and the like; legumes for example, soybean, pea, lentil and the like; root plants for example sugar beets, potatoes, radishes and the like; forages for example alfalfa, clover, grasses and the like; vegetables for example tomato, capsicum, cucumber and the like; turf for example bent grass, Kentucky blue grass and the like. That is, the phosphate solubilizing PGPR may enhance growth of any and all suitable plants having nutrient phosphorus requirement.

Similarly, in some embodiments of the invention, the phosphate solubilizing PGPR are used in a soil environment which has low levels of phosphorus or low levels of plant available phosphorus or highly locked phosphorus. It is of note that, as discussed above, the phosphate solubilizing PGPR can be used in any suitable soil conditions as the presence of the phosphate solubilizing PGPR will promote more efficient usage of phosphorus by plants grown in a soil environment and will thereby promote growth of plants grown in the soil environment. Thus, the above-described PGPR are capable of solubilizing phosphate; promoting P-uptake in plants; capable of alleviating a P-deficiency in plant; and capable of alleviating P-deficiency in P-deficient soil fertilized with P fertilizer. The phosphate solubilizing PGPR may be applied to a soil environment which has been or will be treated with a phosphorus-containing fertilizer, for example, rock phosphate. It is also of note that the phosphate solubilizing PGPR may promote uptake of phosphorus and other macro- and micro-nutrients within the soil environment whether provided by a fertilizer or not.

In other embodiments, the phosphate solubilizing PGPR are in combination with a carrier material. The carrier material may be a liquid, pellet, granular mass, powder, mineral or other similar element or may be a plant seed. Specifically, the phosphate solubilizing PGPR may be coated onto a seed using means known in the art. As way of example, the phosphate solubilizing PGPR may be mixed with peat, clay, perlite, vermiculite, mineral, polymer or agriculturally compatible oil. The phosphate solubilizing PGPR may be freeze-dried to a powder. Alternatively, an aqueous slurry of the phosphate solubilizing PGPR may be dried to a powder at a temperature which does not adversely affect bacterial viability. The microbial powder may be mixed with earth materials, minerals, clay, talc, peat or other agriculturally compatible materials. Also a liquid suspension of the phosphate solubilizing PGPR may be used to coat the seeds or be applied to an absorbent material, for example, a granular material or applied as foliar spray on soil or plants.

As will be appreciated by one of skill in the art, survivability of applied bacterial culture is one of the critical factors for successful colonization of the rhizosphere and rhizoplane that results in plant growth, development and yield. For example, soil and landscape scale variability, nutrient status, and climatic conditions may affect bacterial survivability and crop yield. Hence, bacterial strain XSB375, identified as *Bacillus firmus,* is individually effective at promoting plant growth as described herein. In some embodiments, mixtures of XSB375 and any other PGPR may be used in the embodiments of the invention described herein. It is also of note that some combinations may work better under specific physio-chemical conditions of soil, such as soil pH, growth temperature, time of planting, and crop species. These combinations are within the scope of the invention and routine experimentation in the art.

As will be appreciated by one of skill in the art, the phosphate solubilizing PGPR may be applied with other suitable pesticidal agents, for example, herbicides, fungicides, insecticides, seed treatment chemicals or other PGPR and used in the embodiments described herein. That is, applying the phosphate solubilizing PGPR with other biopesticidal PGPR agents or pesticides may further enhance plant growth. Similarly, combining the above-described phosphate solubilizing PGPR with other plant growth-promoting PGPR may have a synergistic effect in promoting plant growth. It is of note that these combinations may be used in any of the above-described embodiments, for example, for coating seeds or liquid foliar application.

The invention will now be described by way of examples. However, it is to be understood that the examples are for illustrative purposes and the invention is not necessarily limited to the examples.

### EXAMPLE 1 - ISOLATION OF PHOSPHATE SOLUBILIZING RHIZOBACTERIA:

Bacteria were isolated from the canola rhizosphere soil and rhizoplane from Elm Creek, Manitoba by a serial dilution technique and spread plate method. The laboratory basal media used was trypticase soy agar (TSA, 1/10 strength) media and the plates were incubated for 3 days at 28°C. All the rhizobacterial isolates were selected to represent distinct types based on colony morphology that includes colony form, elevation, opacity and pigment production. Rhizobacterial isolates were restreaked on TSA plates, checked for purity and stored on TSA slants at 4°C. Presumptive phosphate solubilizing rhizobacteria were isolated by streaking the isolates on modified PDYA (potato-dextrose yeast extract agar) media plates (de Freitas et al., 1997). The modified media consists of the PDYA media containing freshly precipitated calcium phosphate (i.e. 50 ml sterile 10% (wt/vol) K₂HPO₄ and 100 ml sterile 10% (wt/vol) CaCl₂) were added per litre of sterile PDYA to produce a precipitate of CAHPO₄ (Katznelson and Bose, 1959, Can J Microhiol 5: 79-85).

### PHOSPHATE SOLUBILIZATION TEST:

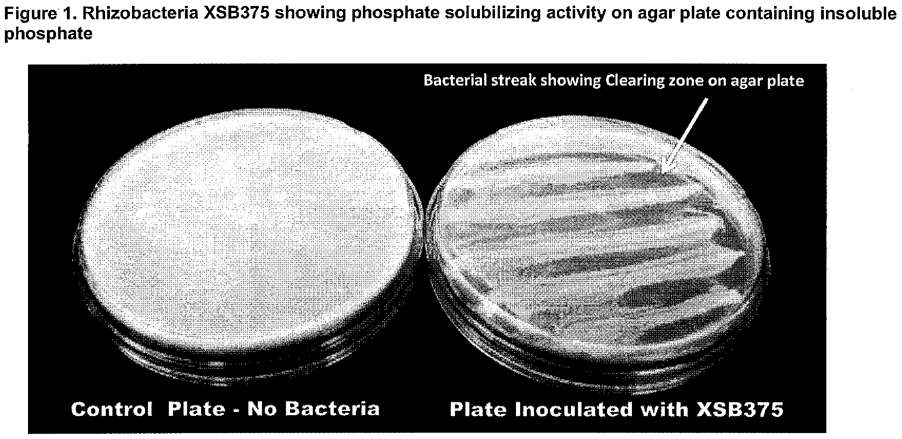

The ability of the rhizobacterial isolates to solubilize phosphate was assessed qualitatively using modified PDYA media (de Freitas et al., 1997). The modified media consists of the PDYA media containing freshly precipitated calcium phosphate (i.e. 50 ml sterile 10% (wt/vol) K₂HPO₄ and 100 ml sterile 10% (wt/vol) CaCl₂) were added per litre of sterile PDYA to produce a precipitate of CAHPO₄ (Katznelson and Bose, 1959). Each rhizobacterial isolate was streaked on PDYA-CaP plate, TSA-CaP plate, and modified TY (tryptone yeast extract)-CaP plate, and incubated at 28°C. Phosphate solubilizing capacity was assessed up to 14 days by measuring the clearing zone (solubilization area) surrounding the bacterial colony. Among the presumptive phosphate solubilizing rhizobacterial strains isolated from canola rhizosphere and rhizoplane bacterial strain XSB375 produced the earliest and largest zone of clearing on phosphate precipitated plates (Figure 1).

### EXAMPLE 2 - INOCULATION EFFECT ON CANOLA SEED EMERGENCE:

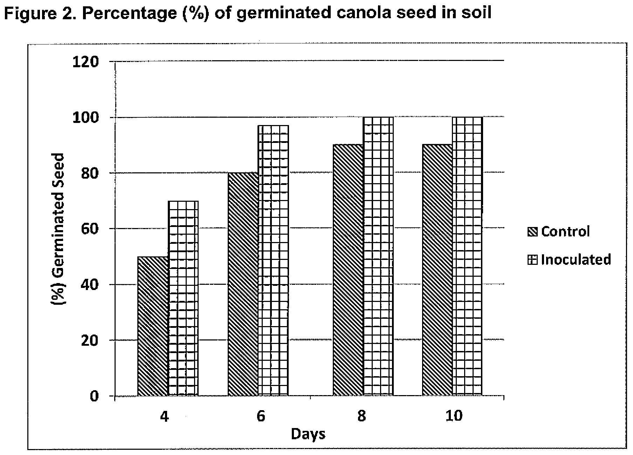

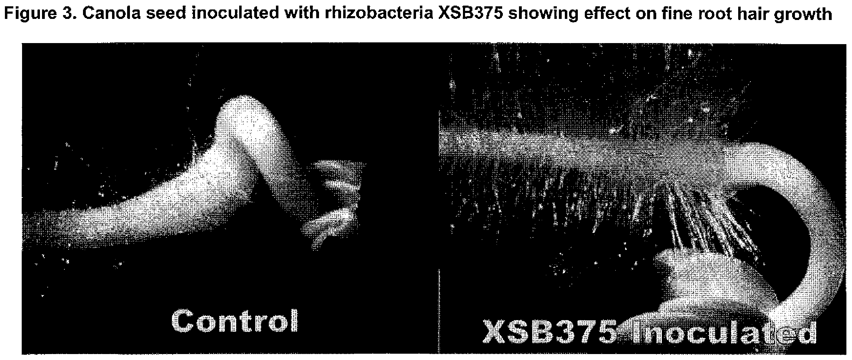

Bacterial strain XSB375 was grown in 100ml of TY broth for 48 hours on a rotary shaker. Bacterial cell culture was concentrated by centrifugation and washed with distilled water and suspended in NaCl solution. Surface disinfected canola seeds were then inoculated with the bacterial suspension. Inoculated seeds were placed on agar plates to observe the bacterial effect on canola seed emergence (Table 1). A set of uninoculated seeds were also placed on agar plates as control. Inoculated seeds were also planted on soil pots and growth pouches to examine bacterial effect in soil (Figure 2) and growth pouch (Table 2). Results illustrated that canola seeds inoculated with strain XSB375 enhanced seed emergence on agar plate, in soil and in growth pouch compared to control (Table 1 and 2, Figure 2 and 3).

### EXAMPLE 3 - INOCULATION EFFECT ON CANOLA GROWTH PROMOTION IN GROWTH POUCH:

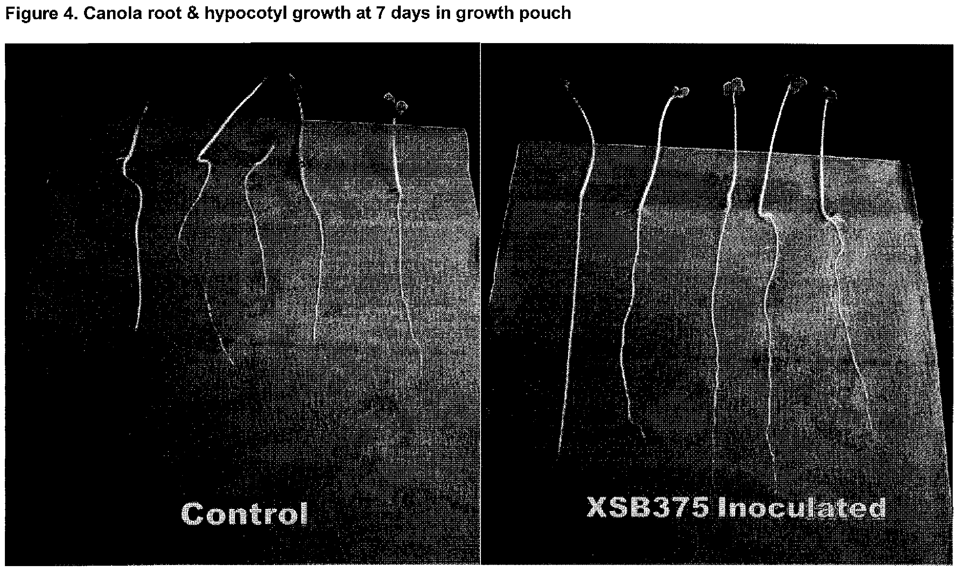

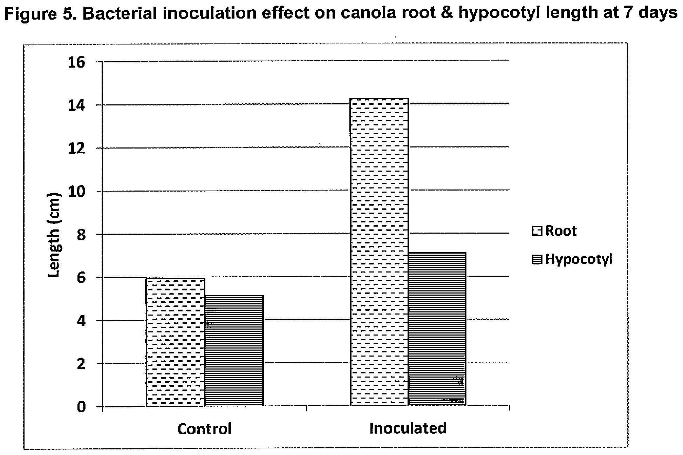

Surface disinfected canola seeds were inoculated with the bacterial suspension. Inoculated seeds were planted in growth pouches to examine the bacterial effect on canola growth promotion (Figure 4). A set of uninoculated seeds were also placed in growth pouch as control. Plant hypocotyl and root lengths were measured after 7 days (Figure 5). Results illustrated that canola seeds inoculated with strain XSB375 enhanced canola growth promotion in growth pouch compared to control (Figure 4 and 5). The bacterial strain XSB375 enhanced the canola root (mean length 14.26 cm) and hypocotyl (mean length 7.11 cm) growth considerably in comparison to control root (5.94 cm) and control hypocotyl (mean length 5.14 cm).

### EXAMPLE 4 - VIABILITY OF BACTERIA ON SEED, IN PEAT POWDER AND IN LIQUID SUSPENSION:

The benefit of microbial inoculation for greater crop production is significantly impacted by the number of live cells introduced into soil (Duquenne et al., 1999). Furthermore, biological activity of microbes may also decline rapidly with handling and storage procedure. It is important to evaluate the duration of bacterial viability in seed treatment or in the carrier materials to obtain the desired level of microbial population for the inoculant to be effective. Seed inoculated with strain XSB375 retained viable cell count well above desired level to 21 days after inoculation (Table 3). Seed coating generally increase the bacterial viability as coating prevents cells from drying out and keeps bacteria live much longer period.

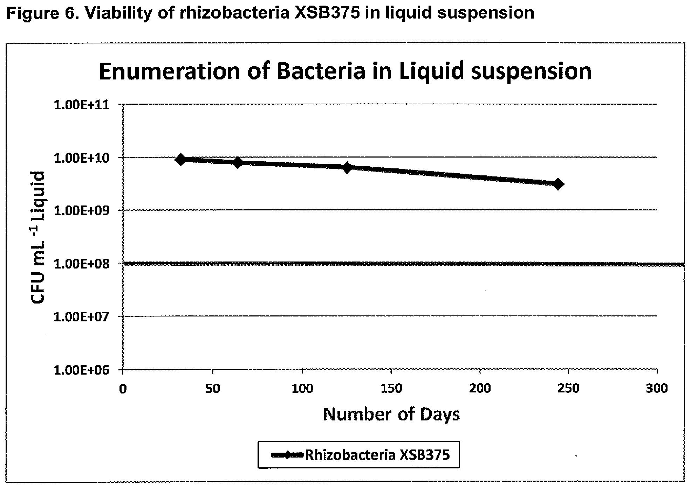

The strain XSB375 was tested for the viability and shelf life with different inoculant carrier materials such as liquid media, peat powder, etc. The bacterial strain XSB375 was grown in modified TY broth for 48 hours and mixed with specifically formulated liquid media used as liquid carrier. The bacteria grown in TY broth was also used directly to inoculate commercially available sterilized peat powder. Bacterial viability was tested periodically for both liquid suspension and peat powder. It is shown that when sterile peat bags were inoculated with the rhizobacterial strain XSB375 survivability of the strain were increased up to 244 days at over 10⁹ cfu per gram of carrier material (Table 4). Moreover, when bacteria XSB375 was inoculated and kept in liquid suspension in a sterile bag and analysis showed that bacterial strain XSB375 remained viable over 240 days and viable bacterial count was more than 1x10⁹ cfu/ml (Figure 6). Both the liquid and peat formulation were found to be effective carrier materials for PGPR strain XSB375 to maintain desired product shelf life.

### EXAMPLE 5 - EFFECT OF PGPR INOCULATION ON CANOLA YIELD IN FIELD TRIALS:

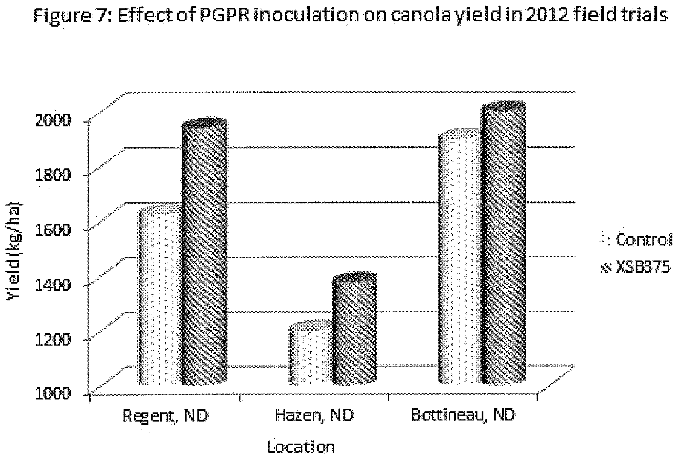

In 2012, three field trials were carried out using PGPR strain XSB375. The canola trials were planted through commercial seed planter and foliar bacterial treatment was applied during first post emergent herbicide application using tank mix. Herbicide tolerant canola cultivar was used and all field trials were split strip design, each strip was size of 10 acres, with one bacterial treatment along with one control, replicated three to four times. The treatments were designed to evaluate bacterial ability to enhance canola yield. Strips were harvested at maturity, seed was collected and cleaned, and yield was measured based on 8.5% seed moisture. Canola yield data in Regent, ND; Hazen, ND; and Bottineau, ND; sites were presented in Figure 7. Figure 7 showed that the PGPR strain XSB375 treatment increased canola yield by 19.4% compared to the control in Regent, ND; 14.6% compared to the control in Hazen, ND; and 6.6% compared the control in Bottineau, ND; whereas, the PGPR XSB375 treatment on an average increased canola yield by 13.5% compared to the uninoculated control across all the sites in North Dakota.

### EXAMPLE 6 - IDENTIFICATION OF RHIZOBACTERIAL STRAIN:

The bacterial strain XSB375 was grown on modified TY media for 48 hours. Then the bacterial DNA was extracted from the bacteria for 16S DNA and 500 bp (base pair) identification using the services of MIDI LABS (Newark, DE). The strain was identified as *Bacillus firmus* XSB375.

**Table 1. Effect of bacterial inoculation on canola seed emergence on agar plate**

| **Treatment** | **Percent (%) Emergence** | | | |
|---|---|---|---|---|
| | **2-Day** | **4-Day** | **6-Day** | **8-Day** |
| Control | 60 | 99 | 99 | 99 |
| Inoculated Seed | 80 | 100 | 100 | 100 |

**Table 2. Effect of bacterial inoculation on canola seed emergence in growth pouch**

| **Treatment** | **Percent (%) Emergence** | | | |
|---|---|---|---|---|
| | **2-Day** | **4-Day** | **6-Day** | **8-Day** |
| Control | 60 | 94 | 99 | 99 |
| Inoculated Seed | 80 | 100 | 100 | 100 |

**Table 3. Viability of rhizobacteria XSB375 in inoculated canola seed**

| **Treatment** | **CFU x 10⁵ per Seed** | | | |
|---|---|---|---|---|
| | **4-Day** | **7-dray** | **14-Day** | **21-dray** |
| Control | 0 | 0 | 0 | 0 |
| Inoculated Seed | 63 | 42 | 34 | 12 |

**Table 4. Viability of rhizobacteria XSB375 in inoculated peat powder**

| **Bacterial Strain** | **Incubation Period (Day)** | **CFU/g of Peat carrier materials** |
|---|---|---|
| XSB375 | 32 | >10⁹ |
| | 64 | >10⁹ |
| | 125 | >10⁹ |
| | 244 | >10⁹ |

## Claims

1. A biologically pure bacterial culture of XSB375 (*Bacillus firmus*) ATTC# PTA-120309.

2. A method of increasing plant growth and/or yield comprising:
inoculating with phosphate solubilizing plant growth promoting rhizobacteria (PGPR) XSB375 (*Bacillus firmus*) ATTC#PTA-120309 in soil environment or potting mixture; and
growing a plant in said soil environment or potting mixture.

3. The method according to claim 2 wherein the soil environment or potting mixture includes phosphorus as a fertilizer.

4. The method according to claim 2 wherein the PGPR promote P-uptake in a plant.

5. The method according to claim 2 wherein the PGPR is capable of solubilizing phosphate to H₂PO₄⁻ or HPO₄²⁻.

6. The method according to claim 2 wherein the PGPR is capable of alleviating a P-deficiency in a plant or soil or P-deficient soil fertilized with P fertilizer.

7. The method according to claim 2 wherein the PGPR promote uptake of macronutrients or micronutrients in a plant.

8. The method according to claim 2 wherein the plant is an oil seed, legume, non- legume, cereal, root plant, vegetable, grass, forage, turf, fruits and flowering plant.

9. The method according to claim 2 wherein the plant is selected from the group consisting of canola, soybean, sunflower, flax, hemp, wheat, barley, corn, millet, oat, pea, lentil, beans, peanut, alfalfa, clover, sugar beets, potatoes, carrots, cabbages, tomatoes, radishes, capsicum, cucumber, grasses, turf, cantaloupe, melon, fruits and flowers.

10. The method according to claim 2 wherein the phosphate solubilizing PGPR is applied to the soil environment as a liquid suspension.

11. The method according to claim 10 wherein the liquid suspension is applied on plant, on post emergent plant, on seed, on seedling, or on carrier materials.

12. The method according to claim 2 wherein the phosphate solubilizing PGPR is applied to the soil environment as a coated seed.

13. A composition of matter comprising one phosphate solubilizing PGPR XSB375 (*Bacillus firmus*) ATTC# PTA-120309; and an agriculturally compatible carrier.

14. The composition of matter according to claim 13 wherein the carrier is a seed.

15. The composition of matter according to claim 14 wherein the phosphate solubilizing PGPR is coated onto the seed.

16. The composition of matter according to claim 15 wherein the seed is coated with peat or clay or mineral or vermiculite or polymer.

17. The composition of matter according to claim 13 wherein the carrier is a liquid suspension.

18. The composition of matter according to claim 17 wherein the carrier is an agriculturally compatible oil.

19. The composition of matter according to claim 13 wherein the carrier is a granular material.

20. The composition of matter according to claim 13 wherein the granular material is peat or clay or perlite granules or mixture thereof.

21. The method according to claim 2 including applying to said soil environment a compatible herbicide or fungicide or insecticide or seed treatment chemical.

22. The method according to claim 2 wherein the PGPR is used as consortia with symbiotic microorganisms or non-symbiotic microorganisms or in combination with other PGPR or in combination with other biocontrol microorganisms or with plant growth promoting substances.

## Patentansprüche

1. Biologisch reine Bakterienkultur von XSB375 (*Bacillus firmus*) ATTC# PTA-120309.

2. Verfahren zur Erhöhung des Pflanzenwachstums und/oder des Pfanzenertrags, umfassend:
Inokulieren von Phosphat solubilisierendem und das Pflanzenwachstum fördernden Rhizobakterien (PGPR) XSB375 (*Bacillus firmus*) ATCC # PTA-120309 in einer Bodenumgebung oder einer Eintopfmischung; und
Anziehen einer Pflanze in der Bodenumgebung oder der Eintopfmischung.

3. Verfahren nach Anspruch 2, wobei die Bodenumgebung oder die Eintopfmischung Phosphor als Düngemittel enthält.

4. Verfahren nach Anspruch 2, wobei das PGPR die Phosphor-Aufnahme in einer Pflanze fördert.

5. Verfahren nach Anspruch 2, wobei das PGPR Phosphat zu H₂PO₄⁻ oder HPO₄²⁻ solubilisieren kann.

6. Verfahren nach Anspruch 2, wobei das PGPR in der Lage ist, einen Phosphor-Mangel in einer Pflanze oder einem Boden oder einem mit Phosphor-Dünger gedüngten Phosphor-Mangel-Boden zu lindern.

7. Verfahren nach Anspruch 2, wobei das PGPR die Aufnahme von Makronährstoffen oder von Mikronährstoffen in einer Pflanze fördert.

8. Verfahren nach Anspruch 2, wobei die Pflanze ein Ölsamen, eine Hülsenfrucht, eine Nicht-Hülsenfrucht, ein Getreide, eine Wurzelpflanze, ein Gemüse, ein Gras, ein Futter, ein Rasen, Früchte oder eine blühende Pflanze ist.

9. Verfahren nach Anspruch 2, wobei die Pflanze ausgewählt ist aus der Gruppe bestehend aus Canola, Sojabohne, Sonnenblume, Flachs, Hanf, Weizen, Gerste, Mais, Hirse, Hafer, Erbse, Linse, Bohnen, Erdnuss, Alfalfa, Klee, Zuckerrüben, Kartoffeln, Karotten, Kohl, Tomaten, Radieschen, Paprika, Gurke, Gräser, Rasen, Cantaloup-Melone, Melone, Früchte und Blumen.

10. Verfahren nach Anspruch 2, wobei das Phosphat solubilisierende PGPR als eine flüssige Suspension auf die Bodenumgebung aufgebracht wird.

11. Verfahren nach Anspruch 10, wobei die flüssige Suspension auf Pflanzen, auf nachwachsenden Pflanzen, auf Samen, auf Sämlingen oder auf Trägermaterialien aufgebracht wird.

12. Verfahren nach Anspruch 2, wobei das Phosphat solubilisierende PGPR als ein ummanteltes Saatgut auf die Bodenumgebung aufgebracht wird.

13. Stoffzusammensetzung, umfassend ein Phosphat solubilisierendes PGPR XSB375 (*Bacillus firmus*) ATCC # PTA-120309; und einen landwirtschaftlich verträglicher Träger.

14. Stoffzusammensetzung nach Anspruch 13, wobei der Träger ein Samen ist.

15. Stoffzusammensetzung nach Anspruch 14, wobei das Phosphat solubilisierende PGPR auf den Samen beschichtet ist.

16. Stoffzusammensetzung nach Anspruch 15, wobei der Samen mit Torf oder Ton oder Mineral oder Vermiculit oder Polymer beschichtet ist.

17. Stoffzusammensetzung nach Anspruch 13, wobei der Träger eine flüssige Suspension ist.

18. Stoffzusammensetzung nach Anspruch 17, wobei der Träger ein landwirtschaftlich verträgliches Öl ist.

19. Stoffzusammensetzung nach Anspruch 13, wobei der Träger ein granulares Material ist.

20. Stoffzusammensetzung nach Anspruch 13, wobei das granulare Material Torf oder Ton oder Perlitgranulat oder eine Mischung davon ist.

21. Verfahren nach Anspruch 2, umfassend das Aufbringen eines kompatiblen Herbizids oder Fungizids oder Insektizids oder einer Saatgutbehandlungschemikalie auf die Bodenumgebung.

22. Verfahren nach Anspruch 2, wobei das PGPR als Konsortium mit symbiotischen Mikroorganismen oder nicht-symbiotischen Mikroorganismen oder in Kombination mit anderen PGPR oder in Kombination mit anderen Mikroorganismen zur biologischen Regelung oder mit das Pflanzenwachstum fördernden Substanzen verwendet wird.

## Revendications

1. Culture bactérienne biologiquement pure de XSB375 (*Bacillus firmus*) ATTC# PTA-120309.

2. Procédé de stimulation de la croissance et /ou du rendement de végétaux, comprenant :
l'inoculation d'une rhizobactérie solubilisant le phosphate et favorisant la croissance des végétaux (PGPR) XSB375 (*Bacillus firmus*) ATTC# PTA-120309 dans l'environnement édaphique ou mélange d'empotage ; et
la culture d'un végétal dans ledit environnement édaphique ou mélange d'empotage.

3. Procédé selon la revendication 2, où l'environnement édaphique ou le mélange d'empotage contient du phosphore en tant que fertilisant.

4. Procédé selon la revendication 2, où la PGPR favorise l'assimilation du phosphore dans un végétal.

5. Procédé selon la revendication 2, où la PGPR peut solubiliser le phosphate en H₂PO₄⁻ ou HPO₄²⁻.

6. Procédé selon la revendication 2, où la PGPR peut atténuer une déficience en P d'un végétal ou d'un sol ou d'un sol déficient en P fertilisé avec un fertilisant P.

7. Procédé selon la revendication 2, où la PGPR favorise l'assimilation de macronutriments ou de micronutriments dans un végétal.

8. Procédé selon la revendication 2, où le végétal est une graine oléagineuse, une légumineuse, une non-légumineuse, une céréale, une plante à racine, un légume, une herbe, un plante fourragère, du gazon, un fruit ou une plante en fleur.

9. Procédé selon la revendication 2, où le végétal est sélectionné dans le groupe comprenant canola, soja, tournesol, lin, chanvre, blé, orge, maïs, millet, avoine, pois, lentille, haricot, arachide, luzerne, trèfle, betterave à sucre, pomme de terre, carotte, chou, tomate, radis, poivron, concombre, graminées, gazon, cantaloup, melon, fruits et fleurs.

10. Procédé selon la revendication 2, où la PGPR solubilisant le phosphate est appliquée dans l'environnement édaphique sous forme de suspension liquide.

11. Procédé selon la revendication 10, où la suspension liquide est appliquée sur un végétal, une plante en post-levée, des graines, des plants, ou des substrats.

12. Procédé selon la revendication 2, où la PGPR solubilisant le phosphate est appliquée dans l'environnement édaphique sous forme de semence enrobée.

13. Composition de matière comprenant une PGPR solubilisant le phosphate XSB375 (*Bacillus firmus*) ATTC# PTA-120309 ; et
un support approprié à l'agriculture.

14. Composition de matière selon la revendication 13, où le support est une graine.

15. Composition de matière selon la revendication 14, où la graine est enrobée de PGPR solubilisant le phosphate.

16. Composition de matière selon la revendication 15, où la graine est enrobée de tourbe, d'argile, d'un minéral, de vermiculite ou d'un polymère.

17. Composition de matière selon la revendication 13, où le support est une suspension liquide.

18. Composition de matière selon la revendication 17, où le support est une huile appropriée à l'agriculture.

19. Composition de matière selon la revendication 13, où le support est une matière granulaire.

20. Composition de matière selon la revendication 13, où la matière granulaire est de la tourbe, de l'argile, des granulés de perlite ou des mélanges de ceux-ci.

21. Procédé selon la revendication 2, comprenant l'application dans l'environnement édaphique d'un compatible herbicide, d'un fongicide, d'un insecticide ou d'un agent chimique de traitement des graines compatibles.

22. Procédé selon la revendication 2, où la PGPR est utilisée sous forme de groupements avec des microorganismes symbiotiques ou des microorganismes non symbiotiques, en combinaison avec d'autres PGPR ou en combinaison avec d'autres microorganismes de biocontrôle ou avec des substances stimulant la croissance des végétaux.
